# EUROPÄISCHE PATENTSCHRIFT

(11) **EP 1 952 762 B1**
(45) Veröffentlichungstag und Bekanntmachung des Hinweises auf die Patenterteilung: **09.12.2009**
(21) Anmeldenummer: 07023000.8
(22) Anmeldetag: 28.11.2007
(51) Int. Cl.: A61B 5/0452, A61B 5/046, A61B 5/0456, A61B 5/0468, A61N 1/39, A61N 1/362, G06F 17/00

(54) **Detektor für atriales Flimmern und Flattern sowie atriale Fibrillation**
Detector for atrial fibrillation and atrial flutter
Détecteur de flutter auriculaire et de fibrillation auriculaire

(30) Priorität: 21.12.2006 DE 102006061988
(43) Veröffentlichungstag der Anmeldung: 06.08.2008
(73) Patentinhaber: Biotronik CRM Patent AG, 6341 Baar (CH); Universität Freiburg, 79106 Freiburg (DE)
(72) Erfinder: Faber, Thomas S., Dr., 79104 Freiburg (DE); Lippert, Michael, Dr., 91522 Ansbach (DE); Schweika-Kresimon, Marc Oliver, Dr., 44625 Herne (DE)
(74) Vertreter: Lindner-Vogt, Karin L.

(56) Entgegenhaltungen:
- EP-A- 1 769 823
- US-A1- 2005 080 347
- US-A1- 2006 167 364

## Beschreibung

Die Erfindung betrifft einen Detektor für atriales Flimmern, auch als atriale Fibrillation bekannt, und atriales Flattern ("AF" steht im Folgenden für atriales Flimmern oder Flattern). Der Detektor ist vorzugsweise Bestandteil eines medizinischen Implantats wie beispielsweise eines implantierbaren Herzschrittmachers oder eines implantierbaren Kardioverters/Defibrillators.

Die atriale Fibrillation ist ein Zustand ungeordneter, das atriale Flattern beispielsweise kreisender Erregung, des atrialen Myokards, bei dem das Atrium praktisch keinen Beitrag zur Pumpleistung eines Herzens leistet. In einem intraatrialen Elektrokardiogramm ist eine atriale Fibrillation an einer hohen Erregungsfrequenz und geringer Amplitude zu erkennen.

Es ist bekannt, eine atriale Defibrillation beispielsweise mit Hilfe implantierbarer atrialer Defibrillatoren zu behandeln. Beispiele für derartige atriale Defibrillatoren und Detektoren für atriale Fibrillation sind in US 5,267,559, US 5,433,729, US 5,464,431, US 5,464,432, US 5,486,199 und US 5,720,295 zu finden.

Aus US 2006/0167364 ist ein Detektor für atriale Fibrillation AF als Bestandteil eines implantierbaren Herzschrittmachers bekannt, der einen atrialen und ventrikulären Signaleingang und entsprechende Elektroden zur Aufnahme eines intraatrialen und eines ventrikulären Elektrokardiogramms aufweist. Mit Hilfe der R-Zacke als Referenzzeitpunkt im ventrikulären Signal wird ein zeitlich verschobener Signalabschnitt aus dem atrialen Signal ausgewählt, über mehrere Signalabschnittel gemittelt und ein Maß für die Signalstärke des gemittelten Signals ermittelt. Der so ermittelte Wert wird mit einem Schwellwert verglichen und bei Unterschreiten zur Erzeugung eines AF-Verdachtssignals herangezogen.

US 2005/0080347 offenbart eine Vorrichtung und eine Methode zur Detektion zur Unterscheidung und Erkennung von Arrhythmien, wobei unter anderem die durchschnittliche atriale bzw. ventrikuläre Zyklenlänge ermittelt, ein Vergleichswert wegen physiologischer und systematischer Veränderlichkeit als Mittelwert der gemessenen Werte mitführt und Abweichungsschwellen als Prozentsatz dieser nachgeführten Größen definiert werden. Weiterhin ist aus dieser Schrift eine Methode zur Unterscheidung verschiedener atrialer Arrhythmien, unter anderem AF und AVB bekannt. Das Dokument zeigt hierzu die Messung der ventrikulären Intervalllänge und offenbart deren Abweichung vom Mittelwert als Kriterium für die Detektion von AVB und zur Auslösung bzw. Anpassung entsprechender Verdachtssignale. Für die Ermittlung und Signalisierung von AVB genügt bereits ein abweichender Zyklus.

Vor dem Hintergrund des bekannten Standes der Technik ist es die Aufgabe der Erfindung, einen Detektor zur zuverlässigen Erkennung von AF zu schaffen, der sich mit vertretbarem Aufwand realisieren lässt und der eine möglichst hohe Sensitivität sowie auch eine möglichst hohe Spezifität besitzt.

Erfindungsgemäß wird diese Aufgabe durch einen Detektor für AF gelöst, der einen atrialen Signaleingang besitzt, der wenigstens indirekt mit einer atrialen Elektrode zur Aufnahme eines intraatrialen Elektrokardiogramms (EKG) verbunden ist oder zu verbinden ist. Der AF Detektor gewinnt aus dem intraatrialen EKG ein atriales Signal, das für jeden, eine atriale Kontraktion und die darauf folgende Entspannung des Atriums umfassenden atrialen Zyklus mehrere, zu verschiedenen Zeitpunkten innerhalb eines jeweiligen atrialen Zyklus erfasste Signalwerte umfasst. Mit anderen Worten: im Betrieb tastet der AF Detektor das intraatriale EKG mit einer Frequenz ab, die größer ist, als die durch die Dauer des atrialen Zyklus (AA-Intervall) bestimmte, atriale Kontraktionsrate.

Neben dem atrialen Signaleingang weist der AF Detektor außerdem einen ventrikulären Signaleingang auf, über den dem AF Detektor ein Ventrikelsignal zuzuführen ist, welches die Zeitpunkte ventrikulärer Kontraktionen in zeitlicher Zuordnung zu dem atrialen Signal widerspiegelt. Ein solches Ventrikelsignal kann ein intraventrikuläres Elektrokardiogramm sein, es kann aber auch ein aus diesem abgeleitetes Signal wie beispielsweise das Signal eines ventrikulären Markerkanals sein. In allen Fällen ist das Ventrikelsignal ein Signal, welches die Zeitpunkte zyklisch wiederkehrender ventrikulärer Ereignisses wie z.B. die Zeitpunkte ventrikulärer Kontraktionen wiedergibt, die sich als R-Zacken im intraventrikulären Elektrokardiogramm widerspiegeln. Für die Erfindung ist es nicht entscheidend, ob das Ventrikelsignal ein Rohsignal ist, wie es unmittelbar durch Erfassen elektrischer Potentiale im Ventrikel gewonnen wird (also mittels ventrikulären EKGs), oder ein daraus abgeleitetes Signal wie das Signal eines Markerkanals ist.

Weiterhin weist der Detektor neben dem atrialen und dem ventrikulären Signaleingang eine Auswerteeinheit auf, die ausgebildet ist,
das atriale Signal in mehrere aufeinander folgende Abschnitte derart aufzuteilen, dass ein jeweiliger Abschnitt des atrialen Signals entweder zu einem Zeitpunkt beginnt, der um eine vorgegebene, vorzugsweise einstellbare Zeitspanne vor einem nächsten ventrikulären Ereignis liegt oder zu einem Zeitpunkt endet, der um eine vorgegebene, vorzugsweise einstellbare Zeitspanne vor einem ventrikulären Ereignis liegt,
die aufeinander folgenden Abschnitte des atrialen Signals miteinander zu mitteln und so ein gemitteltes atriales Signal zu bilden,
die peak-to-peak Amplitude des gemittelten atrialen Signals zu bestimmen,
die peak-to-peak Amplitude des gemittelten atrialen Signals mit einem Vergleichswert zu vergleichen, und
im Falle, dass die peak-to-peak Amplitude des gemittelten atrialen Signals geringer ist, als der Vergleichswert, ein AF-Verdachtsignal zu erzeugen.

Die Auswerteeinheit weist zudem einen Wenckebach-Diskriminator auf, der ausgebildet ist,
auf ein AF-Verdachtssignal anzusprechen und AF von einem AV-Block II Typ Wenckebach zu unterschieden und gegebenenfalls das AF-Verdachtssignal zu löschen,
anhand des Ventrikelsignals einen Mittelwert der Zyklendauer eines ventrikulären Zyklus (RR-Intervall) über eine vorgegebene Anzahl ventrikulärer Zyklen zu bilden, und im Falle des Vorliegens des AF-Verdachtssignals die Dauer eines aktuellen ventrikulären Zyklus mit dem Mittelwert der Zyklendauer zu vergleichen und zu prüfen, ob jeweils nur ein vereinzelter ventrikulärer Zyklus oder mehrere aufeinander folgende ventrikuläre Zyklen hinsichtlich ihrer Dauer um mehr als ein vorgegebenes Differenzmaß von dem Mittelwert der Zyklendauer abweichen und im ersten Fall das AF-Verdachtssignal wieder zu löschen, und
für jeden ventrikulären Zyklus, in dem ein einzelner ventrikulärer Zyklus hinsichtlich seiner Dauer um mehr als ein vorgegebenes Differenzmaß von dem Mittelwert der Zyklendauer abweicht, ein Wenckebach-Signal zu erzeugen.

Die Erfindung zeichnet sich dadauch aus, dass der Wenckebach-Diskriminator einen Zähler aufweist, der ausgebildet ist, seinen Zählerstand um 1 zu erhöhen, falls der Wenckebach-Diskriminator für einen vereinzelten aktuellen ventrikulären Zyklus ein Wenckebach-Signal erzeugt hat.

Der Bezugszeitpunkt für die eine oder die andere Zeitspanne kann auch ein der Zeitspanne unmittelbar vorangehendes ventrikuläres Ereignis sein, so dass der jeweilige Abschnitt des atrialen Signals entweder zu einem Zeitpunkt beginnt, der um eine vorgegebene, vorzugsweise einstellbare Zeitspanne nach einem vorangehenden ventrikulären Ereignis liegt oder zu einem Zeitpunkt endet, der um eine vorgegebene, vorzugsweise einstellbare Zeitspanne nach einem vorangehenden ventrikulären Ereignis liegt.

Das AF-Verdachtssignal kennzeichnet einen atrialen Zustand, in dem der Verdacht auf eine atriale Fibrillation oder atriales Flattern besteht.

Vorzugsweise ist ein jeweiliger atrialer Signalabschnitt ein Abschnitt eines atrialen Signals, der zu einem Zeitpunkt beginnt, der um eine einstellbare Zeitspanne gegenüber einer nachfolgenden R-Zacke im ventrikulären EKG zeitlich versetzt ist und der zu einem Zeitpunkt endet, der um dieselbe Zeitspanne gegenüber einer unmittelbar darauf folgenden R-Zacke zeitlich versetzt ist.

Die Mittelung der atrialen Signalabschnitte über mehrere ventrikuläre Zyklen erfolgt dabei in keiner Weise derart, dass ein jeweiliger Abschnitt des atrialen Signals im Falle unterschiedlich langer ventrikulärer Zyklen gestaucht oder gestreckt wird. Vielmehr werden entweder ein Anfangs- oder ein Endabschnitt eines jeweiligen atrialen Signalabschnittes, der länger ist, als kürzere der zu mittelnden atrialen Signalabschnitte, für die Mittelung außer Betracht gelassen oder Anfangs- bzw. Endabschnitte werden über alle jeweils vorhandenen längeren atrialen Signalabschnitte gemittelt. Alternativ können auch die kürzeren zu mittelnden atrialen Signalabschnitte vor der Mittelung mit Nullen aufgefüllt werden.

Die Erfindung beruht auf der Erkenntnis, dass im Falle von AF atriale Kontraktionen und ventrikuläre Kontraktionen derart dissoziiert sind, dass zwischen den atrialen und den ventrikulären Kontraktionen nicht die bei gesunden Herzen vorhandene Synchronität besteht. Dies führt dazu, dass die atrialen Kontraktionen bezogen auf aufeinander folgende, ventrikuläre Zyklen zu unterschiedlichen Zeitpunkten innerhalb eines jeweiligen ventrikulären Zyklus auftreten. Wenn somit das atriale Signal über mehrere ventrikuläre Zyklen zyklusweise (bezogen auf die ventrikulären Kontraktionen) gemittelt wird, mitteln sich noch vorhandene Spitzenamplituden des atrialen Signals so weitgehend aus, dass das gemittelte atriale Signal keine Spitzenamplituden nennenswerter Größe mehr aufweist. Dieser Zustand wird erfindungsgemäß dadurch erfasst, dass das gemittelte atriale Signal mit einem vorgegebenen, gegebenenfalls automatisch anpassbaren Vergleichswert verglichen wird. Wahlweise kann als Vergleichswert auch ein vorgegebener Prozentsatz des Mittelwerts der peak-to-peak-Amplituden der ungemittelten atrialen Signalabschnitte verwendet werden. Wenn die Spitzenamplitude des gemittelten atrialen Signals kleiner ist, als der Vergleichswert, liegt mit großer Wahrscheinlichkeit AF vor. In diesem Fall wird ein AF-Verdachtssignal erzeugt.

In einer bevorzugten Ausführungsvariante ist die Auswerteeinheit ausgebildet, immer die jeweils acht aktuellsten, zwischen zwei aufeinander folgenden ventrikulären Kontraktionen liegenden Abschnitte des atrialen EKG-Signals miteinander zu mitteln. Es hat sich herausgestellt, dass bereits eine Mittlung über acht ventrikuläre Zyklen zu einer ausreichend signifikanten Reduktion der Spitzenamplitude des gemittelten atrialen Signals führt. Außerdem lässt sich auf diese Weise bereits nach acht ventrikulären Zyklen AF feststellen. Die Mittelung kann auch über mehr als acht ventrikuläre Zyklen erfolgen, wodurch zwar die Spezifität erhöht wird, gleichzeitig aber die Detektionsgeschwindigkeit sinkt.

Zur Bildung des atrialen Signals wird das intraatriale EKG-Signal vorzugsweise mit einer Abtastrate zwischen 30 Hz und 300 Hz abgetastet (gesamplet). 128 Hz ist ein besonders geeigneter Wert.

Um die Spezifität des AF-Detektors zu erhöhen, weist die Auswerteeinheit einen Wenckebach-Diskriminator auf, der ausgebildet ist, auf ein AF-Verdachtssignal anzusprechen und AF von einem AV-Block II° Typ Wenckebach zu unterscheiden und gegebenenfalls ein zuvor gebildetes AF-Verdachtssignal wieder zu löschen. Dies beruht auf der Erkenntnis, dass es im Falle eines AV-Blocks II° vom Typ Wenckebach auch ohne das Vorliegen von AF zum Auslöschen der Spitzenamplituden des atrialen Signals kommen kann, wenn Abschnitte des atrialen Signals über mehrere ventrikuläre Zyklen miteinander gemittelt werden. Dies hängt damit zusammen, dass auch im Falle eines AV-Blocks II° vom Typ Wenckebach die Synchronität zwischen den atrialen Kontraktionen und den ventrikulären Kontraktionen verloren gehen kann. Die Mittlung des atrialen Signals kann also auch im Falle eines AV-Blocks II°, Typ Wenckebach, dazu führen, dass zu Ventrikelkontraktionen asynchrone Anteile des atrialen Signals herausgemittelt werden. Im Falle eines AV-Blocks II° Typ Wenckebach kommt es infolge zunehmender Ermüdung bis zur Erschöpfung der AV-Leitung (der natürlichen Reizüberleitung vom Atrium zum Ventrikel, auch atrio-ventrikuläre Überleitung genannt) dazu, dass die Dauer eines PQ-Intervalls - also die Dauer zwischen einer atrialen Depolarisation (und Kontraktion) bis zu Beginn der ventrikulären Depolarisation und Kontraktion - periodenweise so lange zunimmt, bis eine atrio-ventrikuläre-Überleitung ausfällt. Je nachdem, ob die Zunahme der Dauer des PQ-Intervalls von Herzschlag zu Herzschlag konstant bleibt oder anwächst, werden auch die RR-Intervalle (ein RR-Intervall gibt die Dauer eines ventrikulären Zyklus wieder) über mehrere ventrikuläre Zyklen konstant bleiben oder etwas anwachsen bis eine Überleitung ausfällt. Dadurch ergeben sich periodisch einige ventrikuläre Zyklen mit annähernd konstanten RR-Intervallen und daran anschließend ein relativ lang andauernder ventrikulärer Zyklus mit einem relativ langen RR-Intervall oder, falls ein Herzschrittmacher im Zweikammer-Modus einspringt, einem ventrikulären Zyklus mit relativ kürzerer Zyklusdauer, also kurzem RR-Intervall.

Zur Unterscheidung eines AV-Blocks II° Typ Wenckebach von AF ist daher ein Wenckebach-Diskriminator vorgesehen, der ausgebildet ist, diese Unterscheidung anhand eines Stabilitäts-Kriteriums vorzunehmen. Der Wenckebach-Diskriminator ermittelt über eine Anzahl von N aktuellen ventrikulären Zyklen jeweils kontinuierlich die mittlere Zyklendauer (das mittlere RR-Intervall) RRm oder besser noch den Median-Wert der RR-Intervalle. Liegt immer nur ein ventrikulärer Zyklus hinsichtlich der Dauer seines RR-Intervalls außerhalb eines vorgegebenen Stabilitäts-Bereiches, so ist dies ein Indikator, dass kein AF, sondern ein AV-Block II° Typ Wenckebach vorliegt. Der vorgegebene Stabilitätsbereich ist vorzugsweise durch ein auf den Mittelwert der Zyklendauer bezogenes Differenzmaß derart definiert, dass der Mittelwert der Zyklendauer RRm mit 1 plus/minus dem Differenzmaß multipliziert wird. Der Stabilitätsbereich ergibt sich also durch RRm * (1 +/-d), wobei d das Differenzmaß ist.

Liegen ab und zu auch hintereinander mindestens zwei RR-Intervalle außerhalb des so definierten Stabilitätsbereiches, schließt der Wenckebach-Diskriminator das Vorliegen eines AV-Block II° Typ Wenckebach aus und bestätigt das AF-Verdachtssignal, das heißt der AF-Detektor detektiert dann endgültig AF.

In einer bevorzugten Ausführungsvariante ist der Wenckebach-Diskriminator ausgebildet, in der folgenden Weise zu arbeiten:
Für den Fall, dass die Spitzenamplitude des gemittelten atrialen Signals unter den vorgegebenen Vergleichswert abgesunken ist, das heißt für den Fall, dass die Auswerteeinheit ein AF-Verdachtssignal erzeugt hat, wird der Wenckebach-Diskriminator angewandt, der auf nachfolgende Weise einen Zählerwert bildet. Wahlweise kann dafür der Wenckebach-Diskriminator ständig im Hintergrund aktiv sein, oder nur bei Vorliegen eines AF-Verdachtssignals einen gespeicherten RR-Intervalltrend auswerten. Zunächst bildet der Wenckebach-Diskriminator für jeden ventrikulären Zyklus aktuell den Stabilitätsbereich als RR-Stabilitätsintervall durch Multiplizieren der über die aktuellen N ventrikulären Zyklen gemittelten Dauer der RR-Intervalle RRm (mittlere Zyklendauer der ventrikulären Zyklen) mit zwei Differenzfaktoren und bestimmt so die obere und untere Grenze des jeweils aktuellen RR-Stabilitätsintervalls (des aktuellen Stabilitätsbereiches). Der Differenzfaktor ergibt sich aus der Summe von 1 plus einem vorgegebenen Differenzmaß d, welches zum Beispiel 0,25 beträgt, beziehungsweise 1 minus d. Die über N ventrikuläre Zyklen gemittelte ventrikuläre Zyklendauer kann der Mittelwert der aktuellen N RR-Intervalle sein oder vorzugsweise der Median einer Anzahl N aktueller RR-Intervalle. Alternativ kann die mittlere ventrikuläre Zyklendauer auch durch einen rekursiven Filter mit N als eine Art "Zeitkonstante" gebildet werden. Ein geeigneter Wert für die Anzahl N liegt zwischen 5 und 8.

Der Wenckebach-Diskriminator prüft für jeden ventrikulären Zyklus, ob das entsprechende RR-Intervall innerhalb des auf vorstehende Weise gebildeten RR-Stabilitätsintervalls RRm * (1 +/- d) liegt. Liegt ein jeweils aktuelles RR-Intervall außerhalb dieses aktuell gültigen RR-Stabilitätsintervalls und liegt das darauf folgende RR-Intervall wieder innerhalb des RR-Stabilitätsintervalls, so wird der Zählerstand des Zählers des Wenckebach-Diskriminators um 1 hochgezählt. Der Zählerstand des Zählers des Wenckebach-Diskriminators kann dabei höchstens einen vorgegebenen Maximalwert Nmax von beispielsweise 20 erreichen, das heißt, sobald der Zählerstand 20 erreicht ist, kann dieser Zählerstand nicht weiter hochgezählt werden. Sobald zwei aufeinander folgende ventrikuläre Zyklen RR-Intervalle aufweisen, die außerhalb des RR-Stabilitätsintervalls liegen oder eine Mehrzahl von beispielsweise 20 aufeinander folgenden RR-Intervallen innerhalb des RR-Stabilitätsintervalls liegen, wird der Zähler des Wenckebach-Diskriminators wieder um 1 verkleinert. Erreicht der Zählerstand des Zählers des Wenckebach-Diskriminators eine vorgegebene Zählerstandsschwelle Nth von beispielsweise 10, detektiert der Wenckebach-Diskriminiator das Vorliegen eines AV-Block II° Typ Wenckebach und löscht das AF-Verdachtsignal. Auf diese Weise wird vermieden, dass beispielsweise eine atriale Defibrillation ausgelöst wird, obwohl keine atriale Fibrillation, sondern ein AV-Block II° Typ Wenckebach vorliegt.

Anstelle die Stabilität der RR-Intervalle auf die vorbeschriebene Art und Weise auszuwerten, kann der Wenckebach-Diskriminator auch ausgebildet sein, die Stabilität der Rate der ventrikulären Kontraktionen auszuwerten.

Die Erfindung soll nun anhand eines Ausführungsbeispiels mit Bezug auf die beigefügten Figuren näher erläutert werden. Von den Figuren zeigen:
- Fig. 1:: einen Zweikammer-Herzschrittmacher mit daran angeschlossener und im Herzen platzierter ventrikulärer und atrialer Elektrodenleitung;
- Fig. 2:: ein schematisches Blockschaltbild des Herzschrittmachers aus Fig. 1 mit einem erfindungsgemäßen AF-Detektor;
- Fig. 3:: ein Flussdiagramm eines erfindungsgemäßen Verfahrens zum Detektieren von AF.

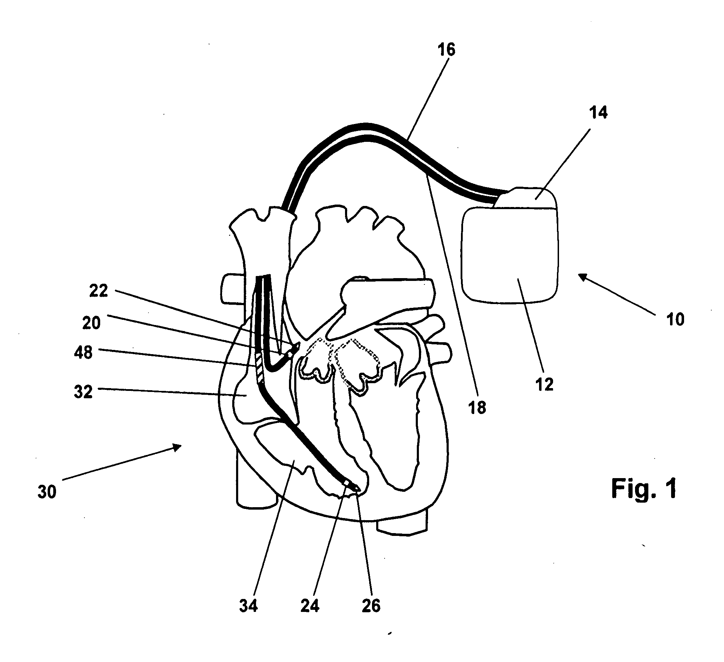

In Fig. 1 ist ein implantierbarer Herzschrittmacher 10 abgebildet, der ein Hohlgehäuse 12 aus Metall besitzt sowie einen Header 14 aus transparentem, isolierendem Kunststoff, in dem Anschlüsse für Elektrodenleitungen angeordnet sind. An den Herzschrittmacher 10 sind eine atriale Elektrodenleitung 16 und eine ventrikuläre Elektrodenleitung 18 angeschlossen.

Die atriale Elektrodenleitung 16 trägt im Bereich ihres distalen Endes eine atriale Ringelektrode 20 und eine atriale Tipelektrode 22.

Die ventrikuläre Elektrodenleitung 18 trägt an ihrem distalen Ende eine ventrikuläre Ringelektrode 24 und eine ventrikuläre Tipelektrode 26.

Wie Fig. 1 zu entnehmen ist, sind die distalen Enden der atrialen Elektrodenleitung 16 und der ventrikulären Elektrodenleitung 18 im Betrieb des Herzschrittmachers 10 in einem Herzen 30 platziert, und zwar derart, dass sich die atriale Ringelektrode 20 und die atriale Tipelektrode 22 im rechten Atrium 32 des Herzens 30 befinden, während die ventrikuläre Ringelektrode 24 und die ventrikuläre Tipelektrode 26 im Apex eines rechten Ventrikels 34 des Herzens 30 angeordnet sind. Für die Erfindung ist es von Vorteil, wenn wenigstens die atriale Tipelektrode 22 wandständig ist, das heißt direkt am Herzmuskelgewebe (Myokard) im Atrium 32 anliegt.

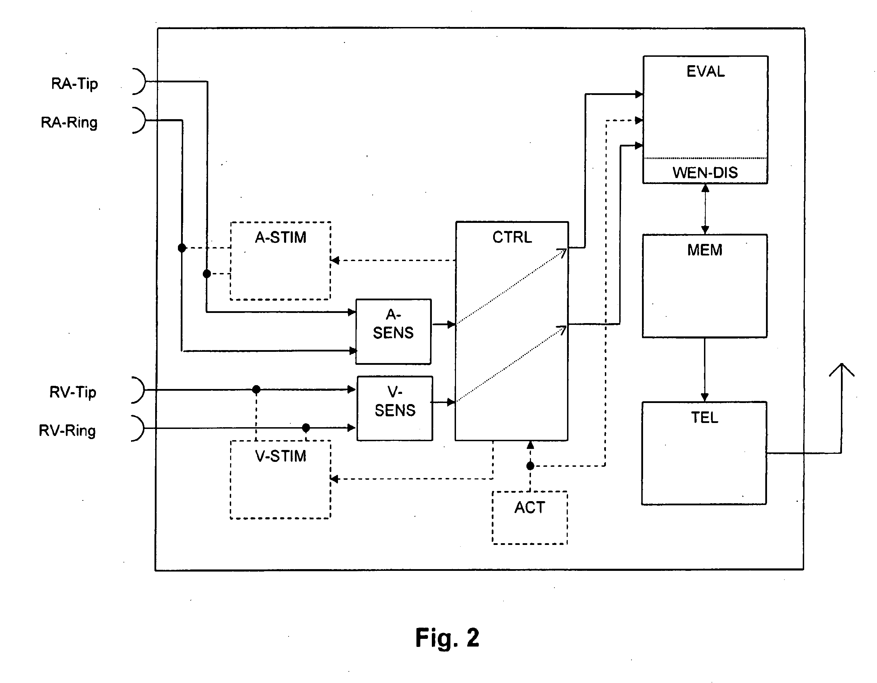

Im Inneren des Hohlgehäuses 12 des Herzschrittmachers 10 sind die wesentlichen elektrischen Komponenten des Herzschrittmachers angeordnet und über den Header 14 mit Anschlüssen für die Elektroden der atrialen Elektrodenleitung 16 und der ventrikulären Elektrodenleitung 18 elektrisch verbunden. In Fig. 2 dargestellt sind ein elektrischer Anschluss RA-Ring für die rechtsatriale Ringelektrode 20, ein weiterer elektrischer Anschluss RA-Tip für die rechtsatriale Ringelektrode 22, ein elektrischer Anschluss RV-Ring für die rechtsventrikuläre Ringelektrode 24 und ein elektrischer Anschluss RV-Tip für die rechtsventrikuläre Tipelektrode 26.

In einer für einen Zweikammer-Herzschrittmacher üblichen Manier sind die Anschlüsse RA-Ring und RA-Tip mit einer atrialen Stimulationseinheit A-STIM und einer atrialen Sensing-Einheit A-SENS und die Anschlüsse RV-Ring und RV-Tip mit einer ventrikulären Stimulationseinheit V-STIM und einer ventrikulären Sensing-Einheit V-SENS verbunden. Die Stimulations- und Sensing-Einheiten sind jeweils mit einer zentralen Steuereinheit CTRL verbunden und erlauben mit dieser zusammen die bedarfsabhängige Stimulation in einem Demand-Modus wie beispielsweise DDD.

Für die vorliegende Erfindung von vorrangiger Bedeutung ist ein AF-Detektor für die Detektion von atrialer Fibrillation oder atrialem Flattern (AF). Der AF-Detektor besitzt einen atrialen Signaleingang, der im abgebildeten Fall indirekt, nämlich über die Steuereinheit CTRL, mit der atrialen Sensing-Einheit A-SENS verbunden ist und auf diesem Wege eine intraatriales EKG-Signal als atriales Signal empfängt. Außerdem besitzt der AF-Detektor einen ventrikulären Signaleingang, der im abgebildeten Fall indirekt, nämlich ebenfalls über die Steuereinheit CTRL, mit der ventrikulären Sensing-Einheit V-SENS verbunden ist und auf diesem Wege eine intraventrikuläres EKG-Signal oder ventrikuläre Markersignale empfängt.

Weiterer Bestandteil des AF-Detektors ist eine Auswerteeinheit EVAL die ihrerseits als Bestandteil einen Wenckebach-Diskriminator WEN-DIS aufweist. Nicht näher dargestellt ist ein Zähler als Bestandteil des Wenckebach-Diskriminators WEN-DIS.

Das atriale Signal ist mit einer Frequenz von 128 zeitabgetastet (gesampelt) und umfasst damit mehrere Signalwerte pro atrialem Zyklus.

In der Auswerteeinheit EVAL wird das atriale Signal in der zuvor beschriebenen Weise ausgewertet. Um diese Auswertung vornehmen zu können, wird der Auswerteeinheit EVAL außerdem ein Ventrikelsignal zugeführt. Dieses Ventrikelsignal repräsentiert die Zeitpunkte ventrikulärer Kontraktionen in zeitlicher Zuordnung zu dem atrialen Signal.

Das Ventrikelsignal wird in an sich bekannter Weise mit Hilfe der Steuereinheit CTRL des Herzschrittmachers gebildet, die mit der ventrikulären Sensingstufe V-SENS verbunden ist. Die ventrikuläre Sensingstufe ist ihrerseits im Betrieb mit der ventrikulären Ringelektrode 24 und der ventrikulären Tipelektrode 26 über entsprechende Anschlüsse RV-Ring und RV-Tip des Herzschrittmachers 10 verbunden.

Wie eingangs bereits beschrieben, bildet die Auswerteeinheit EVAL zwischen jeweils zwei aufeinander folgenden ventrikulären Kontraktionen liegende Signalabschnitte des atrialen Signals und mittelt diese Signalabschnitte über jeweils acht aktuelle ventrikuläre Zyklen und bildet so ein gemitteltes atriales Signal.

Bei gesundem Herzen erfolgt eine jeweilige ventrikuläre Kontraktion nach einer relativ konstanten atrio-ventrikulären Überleitungszeit auf eine atriale Kontraktion, so dass eine Synchronität zwischen atrialen und ventrikulären Kontraktionen besteht. Das auf die vorbeschriebene Art und Weise gemittelte atriale Signal gibt in diesem gesunden Fall den typischen Verlauf des atrialen EKGs zwischen zwei aufeinander folgenden ventrikulären Kontraktionen wieder und besitzt ein Amplitudenmaximum zu einem Zeitpunkt, an dem die atriale Kontraktion stattfindet. Dieser Zeitpunkt liegt vor einer jeweiligen, darauf folgenden ventrikulären Kontraktion und hat zu dieser einen zeitlichen Abstand, der in etwa der atrio-ventrikulären Überleitungszeit entspricht.

Im Falle von AF, aber auch im Falle eines AV-Blocks II° vom Typ Wenckebach besteht die Synchronität zwischen atrialen Kontraktionen und ventrikulären Kontraktionen nicht. Dies führt dazu, dass sich die typischen Maximalamplituden des Verlaufs des atrialen Signals zwischen zwei ventrikulären Kontraktionen bei Mittlung über mehrere ventrikuläre Zyklen ausmitteln, da sie in Bezug auf einen jeweiligen ventrikulären Zyklus zu unterschiedlichen Zeitpunkten auftreten.

Die Erfindung macht sich diesen Sachverhalt zunutze, indem die Auswerteeinheit EVAL eine nicht vorhandene, atrio-ventrikuläre Synchronität durch Auswerten des gemittelten atrialen Signals detektiert. Hierzu wird die Spitzenamplitude des gemittelten atrialen Signals mit einem Vergleichswert verglichen und eine nicht vorhandene atrio-ventrikuläre Synchronität dann detektiert, wenn die Spitzenamplitude des gemittelten atrialen Signals geringer ist als der Vergleichswert.

Da eine nicht vorhandene atrio-ventrikuläre Synchronität ihre Ursache auch in einem AV-Block II° Typ Wenckebach haben kann, besitzt die Auswerteeinheit EVAL außerdem noch einen Wenckebach-Diskriminator WEN-DIS, der ausgebildet ist, auf die eingangs beschriebene Art und Weise das Vorhandensein eines AV-Blocks II° vom Typ Wenckebach zu erfassen, falls die Auswerteeinheit EVAL zunächst eine mangelnde atrio-ventrikuläre Synchronität festgestellt und daraufhin ein AV-Verdachtssignal erzeugt hat. Der Wenckebach-Diskriminator WEN-DIS wirkt als eine Art Filter im Ausgang der Auswerteeinheit EVAL und sorgt dafür, dass die Auswerteeinheit EVAL nur dann ein AF-Verdachtssignal ausgibt, wenn die Auswerteeinheit zum einen die mangelnde atrio-ventrikuläre Synchronität festgestellt und der Wenckebach-Diskriminator zum anderen das Nichtvorhandensein eines AV-Blocks II° vom Typ Wenckebach festgestellt hat.

Für langzeitdiagnostische Zwecke werden die Phasen des Vorliegens eines AF-Verdachtssignals in einem Speicher MEM gespeichert und können mittels einer Telemetrieeinheit TEL drahtlos beispielsweise an ein Service Center übermittelt werden.

Darüber hinaus besitzt der Herzschrittmacher die üblichen Bestandteile zur ratenadaptiven Stimulation des Ventrikels und des Atriums wie beispielsweise die Stimulationseinheiten V-STIM und A-STIM, die Sensing-Einheiten V-SENS und A-SENS, die Steuereinheit CTRL und einen Aktivitätssensor ACT, der es erlaubt, die jeweilige Stimulationsrate an den physiologischen Bedarf eines Patienten anzupassen.

Außerdem kann der Herzschrittmacher auch als Kardioverter/Defibrillator insbesondere als atrialer Defibrillator ausgebildet sein und solche für diesen Zweck angepasste atriale Stimulationseinheiten aufweisen, wie sie aus dem Stand der Technik grundsätzlich bekannt sind.

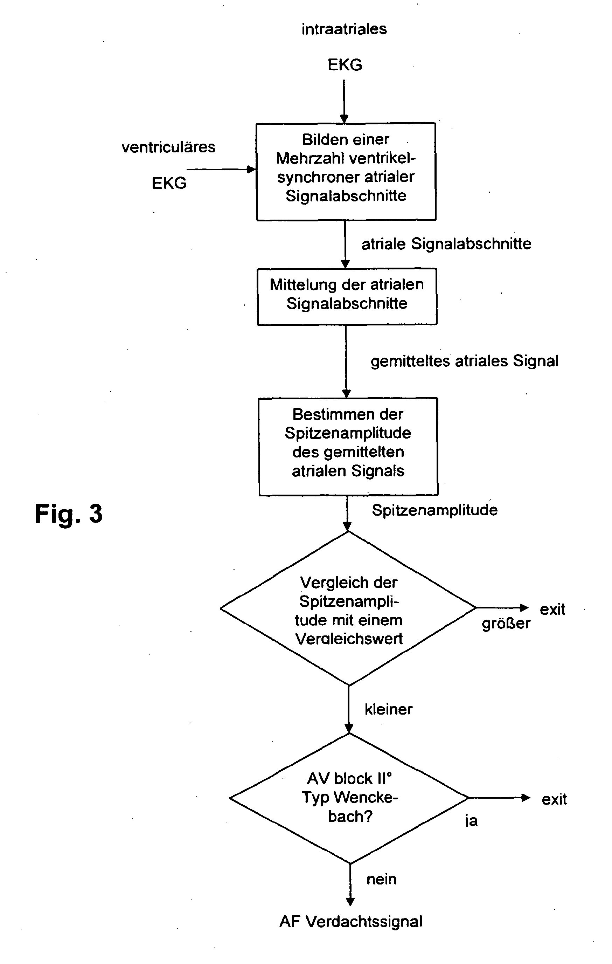

Fig. 3 zeigt die verschiedenen Verfahrensschritte, die der AF-Detektor zum Ermitteln einer atrialen Fibrillation oder eines atrialen Flatterns vornimmt, um ggf. ein AF-Verdachtssignal zu erzeugen.

## Patentansprüche

1. AF-Detektor für atriales Flimmern oder Flattern (AF) mit
einem atrialen Signaleingang, der wenigstens indirekt mit einer atrialen Elektrode zur Aufnahme eines intraatrialen EKG-Signals verbunden ist oder zu verbinden ist, wobei das intraatriale EKG Signal ein atriales Signal bildet, das für jeden, eine atriale Kontraktion und die darauf folgende Entspannung des Atriums umfassenden atrialen Zyklus mehrere, zu verschiedenen Zeitpunkten innerhalb eines jeweiligen atrialen Zyklus erfasste Signalwerte umfasst,
einen ventrikulären Signaleingang, über den dem AF-Detektor ein Ventrikelsignal zuzuführen ist, welches die Zeitpunkte zyklisch wiederkehrender ventrikulärer Ereignisse in zeitlicher Zuordnung zu dem atrialen Signal widerspiegelt, und
einer Auswerteeinheit, die einen Wenckebach-Diskriminator aufweist, der ausgebildet ist,
auf ein AF-Verdachtssignal anzusprechen und AF von einem AV-Block II Typ Wenckebach zu unterschieden und gegebenenfalls das AF-Verdachtssignal zu löschen,
anhand des Ventrikelsignals einen Mittelwert der Zyklendauer eines ventrikulären Zyklus (RR-Intervall) über eine vorgegebene Anzahl ventrikulärer Zyklen zu bilden, und im Falle des Vorliegens des AF-Verdachtssignals die Dauer eines aktuellen ventrikulären Zyklus mit dem Mittelwert der Zyklendauer zu vergleichen und zu prüfen, ob jeweils nur ein vereinzelter ventrikulärer Zyklus oder mehrere aufeinander folgende ventrikuläre Zyklen hinsichtlich ihrer Dauer um mehr als ein vorgegebenes Differenzmaß von dem Mittelwert der Zyklendauer abweichen und im ersten Fall das AF-Verdachtssignal wieder zu löschen, und
für jeden ventrikulären Zyklus, in dem ein einzelner ventrikulärer Zyklus hinsichtlich seiner Dauer um mehr als ein vorgegebenes Differenzmaß von dem Mittelwert der Zyklendauer abweicht, ein Wenckebach-Signal zu erzeugen,
wobei die Auswerteeinheit ausgebildet ist,
das atriale Signal in mehrere aufeinander folgende Abschnitte derart aufzuteilen, dass ein jeweiliger Abschnitt des atrialen Signals entweder zu einem Zeitpunkt beginnt, der um eine vorgegebene Zeitspanne zeitlich versetzt vor einem nächsten ventrikulären Ereignis liegt oder zu einem Zeitpunkt endet, der um eine vorgegebene Zeitspanne zeitlich versetzt vor einem ventrikulären Ereignis liegt,
die aufeinander folgenden Abschnitte des atrialen Signals miteinander zu mitteln und so ein gemitteltes atriales Signal zu bilden,
die peak-to-peak Amplitude des gemittelten atrialen Signals zu bestimmen,
die peak-to-peak Amplitude des gemittelten atrialen Signals mit einem Vergleichswert zu vergleichen, und
im Falle, dass die peak-to-peak Amplitude des gemittelten atrialen Signals geringer ist, als der Vergleichswert, ein AF-Verdachtsignal zu erzeugen,
wobei
der Wenckebach-Diskriminator einen Zähler aufweist, der ausgebildet ist, seinen Zählerstand um 1 zu erhöhen, falls der Wenckebach-Diskriminator für einen vereinzelten aktuellen ventrikulären Zyklus ein Wenckebach-Signal erzeugt hat.

2. AF-Detektor nach Anspruch 1, wobei die vorgegebene Zeitspanne einstellbar ist.

3. AF-Detektor nach Anspruch 1 oder 2, wobei die Auswerteeinheit ausgebildet ist, einen jeweiligen atrialen Signalabschnitt derart zu bilden, dass der atriale Signalabschnitt ein Abschnitt eines intraatrialen EKGs ist, der zu einem Zeitpunkt beginnt, der um eine einstellbare Zeitspanne gegenüber einer nachfolgenden R-Zacke im ventrikulären EKG zeitlich versetzt ist und der zu einem Zeitpunkt endet, der um dieselbe Zeitspanne gegenüber einer unmittelbar darauf folgenden R-Zacke zeitlich versetzt ist.

4. AF-Detektor nach einem der Ansprüche 1 bis 3,
wobei der Vegleideswert vorgegeben und gegebenenfalls automatisch anpassbar ist.

5. AF-Detektor nach Anspruch 4, wobei die Auswerteeinheit ausgebildet ist, den Vergleichswert jeweils derart zu bestimmen, dass der Vergleichswert ein vorgegebener Prozentsatz des Mittelwerts der peak-to-peak Amplituden mehrer ungemittelter, vorangehender und unmittelbar aufeinander folgender Abschnitte des atrialen Signals ist.

6. AF-Detektor nach einem der Ansprüche 1 bis 5,
wobei die Auswerteeinheit ausgebildet ist, immer die jeweils acht aktuellsten Signalabschnitte miteinander zu mitteln.

7. AF-Detektor nach einem der Ansprüche 1 bis 6,
wobei die Auswerteeinheit ausgebildet ist, das intraatriale EKG mit einer Abtastrate zwischen 30Hz und 300Hz abzutasten.

8. AF-Detektor nach Anspruch 1, wobei der Wenckebach-Diskriminator ausgebildet ist, das Differenzmaß aus einem über eine vorgegebene Anzahl aktueller ventrikulärer Zyklen gebildeten Mittelwert der Zyklendauer ventrikulärer Zyklen durch Multiplizieren dieses Mittelwertes mit einem vorgegebenen konstanten Faktor zu bilden.

9. AF-Detektor nach Anspruch 1, wobei der Zähler ausgebildet ist, seinen Zählerstand um 1 zu verringern, falls der Wenckebach-Diskriminator detektiert hat, dass zwei aktuelle, aufeinanderfolgende ventrikuläre Zyklen um mehr als ein vorgegebenes Differenzmaß von dem Mittelwert der Zyklendauer abweichen oder für eine Mehrzahl von wenigstens fünf aufeinander folgenden, aktuellen ventrikulären Zyklen kein Wenckebach-Signal erzeugt hat.

10. AF-Detektor nach Anspruch 9, wobei die Auswerteeinheit ausgebildet ist, das AF-Verdachtssignal zu löschen und den Zähler zurückzusetzen, wenn der Zählerstand eine vorgegebene Zählerstandschwelle überschreitet.

11. AF-Detektor nach einem der Ansprüche 1 bis 10,
wobei der AF Detektor Bestandteil eines implantierbaren medizinischen Gerätes ist.

12. AF-Detektor nach Anspruch 11, wobei das implantierbare medizinische Gerät ein Herzschrittmacher oder ein Kardioverter/Defibrillator ist.

## Claims

1. An AF detector for atrial fibrillation or flutter (AF), comprising
an atrial signal input, which is connected or connectable at least indirectly to an atrial electrode for recording an intra-atrial ECG signal, such that the intra-atrial ECG signal forms an atrial signal comprising several signal values detected at different points in time within a respective atrial cycle for each atrial cycle comprising an atrial contraction and the subsequent relaxation of the atrium,
a ventricular signal input, by means of which a ventricular signal which reflects the points in time of cyclically recurring ventricular events in chronological correlation with the atrial signal, is to be sent to the AF detector, and
an evaluation unit which has a Wenckebach discriminator and is designed
to respond to a suspect AF signal and to differentiate an AF from a second-degree AV block of the Wenckebach type and to delete the suspect AF signal, if necessary,
to form an average cycle time of a ventricular cycle (RR interval) over a predefined number of ventricular cycles on the basis of the ventricular signal and, in the event of occurrence of the suspect AF signal, to compare the duration of a prevailing ventricular cycle with the average cycle time and to check on whether only one isolated ventricular cycle or several successive ventricular cycles deviate from the average cycle duration with regard to their duration by more than a predefined difference, and in the former case to delete the suspect AF signal, and
to generate a Wenckebach signal for each ventricular cycle in which an individual ventricular cycle deviates with regard to its duration by more than a predefined difference from the average of the cycle duration,
whereby the evaluation unit is designed
to divide the atrial signal into several successive portions, such that a respective portion of the atrial signal begins either at a point in time which is offset by a predetermined period of time before the next ventricular event or ends at a point in time which is offset by a predetermined period of time before a ventricular event,
to average the successive portions of the atrial signal and thereby form an averaged atrial signal,
to determine the peak-to-peak amplitude of the averaged atrial signal,
to compare the peak-to-peak amplitude of the averaged atrial signal with a comparative value, and
in the case when the peak-to-peak amplitude of the averaged atrial signal is lower than the comparative value, to generate a suspect AF signal,
wherein
the Wenckebach discriminator has a counter, which is designed to increment its counter reading by one if the Wenckebach discriminator has generated a Wenckebach signal for an isolated prevailing ventricular cycle.

2. The AF detector according to claim 1, wherein the predefined period of time is adjustable.

3. The AF detector according to claim 1 or 2, wherein the evaluation unit is designed to form a respective atrial signal portion, such that the atrial signal portion is a portion of an intra-atrial ECG, which begins at a point in time that is offset by an adjustable period of time in comparison with a following R wave in the ventricular ECG and ends at a point in time which is offset by the same period of time in comparison with a directly following R wave.

4. The AF detector according to any one of claims 1 to 3, wherein the reference value is predefined and is automatically adjustable, if necessary.

5. The AF detector according to claim 4, wherein the evaluation unit is designed to determine the comparative value, such that the comparative value is a predetermined percentage of the average of the peak-to-peak amplitudes of several unaveraged preceding and immediately following portions of the atrial signal.

6. The AF detector according to any one of claims 1 to 5, wherein the evaluation unit is designed to always average the eight most recent signal portions together.

7. The AF detector according to any one of claims 1 to 6, wherein the evaluation unit is designed to sample the intra-atrial ECG with a sampling rate between 30 Hz and 300 Hz.

8. The AF detector according to claim 1, wherein the Wenckebach discriminator is designed to form the difference from an average cycle time of ventricular cycles formed over a predetermined number of prevailing ventricular cycles by multiplying this average cycle time with a predefined constant factor.

9. The AF detector according to claim 1, wherein the counter is designed to decrement its counter reading by 1 if the Wenckebach discriminator has detected that two prevailing successive ventricular cycles deviate by more than a predefined difference from the average of the cycle duration or has not generated a Wenckebach signal for a plurality of at least five successive prevailing ventricular cycles.

10. The AF detector according to claim 9, wherein the evaluation unit is designed to delete the suspect AF signal and to reset the counter when the counter reading exceeds a predefined counter reading threshold.

11. The AF detector according to any one of claims 1 to 10, wherein the AF detector is a component of an implantable medical device.

12. The AF detector according to claim 11, wherein the implantable medical device is a cardiac pacemaker or a cardioverter/defibrillator.

## Revendications

1. Détecteur d'audiofréquence pour scintillement ou vacillement atrial (audiofréquence) comprenant
une entrée de signal atriale, qui est reliée ou peut être reliée au moins indirectement à une électrode atriale pour l'enregistrement d'un signal d'électrocardiogramme intra atrial, le signal d'électrocardiogramme intra atrial formant un signal atrial qui comporte pour chaque cycle atrial comprenant une contraction atriale et la détente consécutive de l'atrium plusieurs valeurs de signal enregistrées à différents moments au sein d'un cycle atrial respectif,
une entrée de signal ventriculaire, par laquelle un signal de ventricule doit être amené au détecteur d'audiofréquence, lequel signal reflète les moments d'évènements ventriculaires récurrents de façon cyclique dans une attribution temporelle au signal atrial, et
une unité d'analyse qui présente un discriminateur de Wenckebach qui est conçu
pour réagir à un signal de suspicion d'audiofréquence et différencier l'audiofréquence d'un bloc AV II de type Wenckebach et éventuellement effacer le signal de suspicion d'audiofréquence,
pour former à l'aide du signal de ventricule une valeur moyenne de la durée de cycle d'un cycle ventriculaire (intervalle RR) au moyen d'un nombre prédéfini de cycles ventriculaires, et, en cas de présence du signal de suspicion d'audiofréquence, comparer la durée d'un cycle ventriculaire actuel avec la valeur moyenne de la durée de cycle et vérifier si à chaque fois uniquement un cycle ventriculaire séparé ou plusieurs cycles ventriculaires consécutifs s'écartent en ce qui concerne leur durée de plus d'une cote de différence prédéfinie de la valeur moyenne de la durée de cycle et, dans le premier cas, effacer à nouveau le signal de suspicion d'audiofréquence et,
générer un signal de Wenckebach pour chaque cycle ventriculaire, dans lequel un cycle ventriculaire individuel diffère en ce qui concerne sa durée de plus d'une cote de différence prédéfinie de la valeur moyenne de la durée de cycle, et
l'unité d'analyse étant conçue
pour diviser le signal atrial en plusieurs parties consécutives de telle sorte qu'une partie respective du signal atrial soit commence à un moment qui est décalé dans le temps d'un laps de temps prédéfini avant un prochain événement ventriculaire soit se termine à un moment qui est décalé dans le temps d'un laps de temps prédéfini avant un événement ventriculaire,
pour calculer la moyenne des parties consécutives du signal atrial et former ainsi un signal atrial moyen,
pour déterminer l'amplitude de crête à crête du signal atrial moyen,
pour comparer l'amplitude de crête à crête du signal atrial moyen avec une valeur de comparaison, et,
au cas où l'amplitude de crête à crête du signal atrial moyen est plus faible que la valeur de référence, pour générer un signal de suspicion d'audiofréquence,
le discriminateur de Wenckebach présentant un compteur qui est conçu pour augmenter son indication de compteur de 1 dans le cas où le discriminateur de Wenckebach a généré un signal de Wenckebach pour un cycle ventriculaire actuel séparé.

2. Détecteur d'audiofréquence selon la revendication 1, le laps de temps prédéfini étant réglable.

3. Détecteur d'audiofréquence selon la revendication 1 ou 2, l'unité d'analyse étant conçue pour former une partie de signal atrial respective de telle sorte que la partie de signal atriale est une partie d'un électrocardiogramme intra atrial qui commence à un moment qui est décalé d'un laps de temps réglable par rapport à un pic R consécutif dans l'électrocardiogramme ventriculaire et qui se termine à un moment qui est décalé dans le temps du même laps de temps par rapport à un pic R consécutif.

4. Détecteur d'audiofréquence selon l'une quelconque des revendications 1 à 3, la valeur de comparaison étant prédéfinie et étant éventuellement adaptable automatiquement.

5. Détecteur d'audiofréquence selon la revendication 4, l'unité d'analyse étant conçue pour déterminer la valeur de comparaison à chaque fois de telle sorte que la valeur de comparaison est un pourcentage prédéfini de la valeur moyenne des amplitudes de crête à crête de plusieurs parties du signal atrial, dont on n'a pas calculé la moyenne, antérieures et directement consécutives.

6. Détecteur d'audiofréquence selon l'une quelconque des revendications 1 à 5, l'unité d'analyse étant conçue pour déterminer toujours la moyenne des huit parties de signal respectivement les plus actuelles les unes avec les autres.

7. Détecteur d'audiofréquence selon l'une quelconque des revendications 1 à 6, l'unité d'analyse étant conçue pour balayer l'électrocardiogramme intra atrial avec un taux de balayage compris entre 30 Hz et 300 Hz.

8. Détecteur d'audiofréquence selon la revendication 1, le discriminateur de Wenckebach étant conçu pour former la cote de différence à partir d'une valeur moyenne, formée au moyen d'un nombre prédéfini de cycles ventriculaires actuels, de la durée de cycle de cycles ventriculaires par multiplication de cette valeur moyenne avec un facteur constant prédéfini.

9. Détecteur d'audiofréquence selon la revendication 1, le compteur étant conçu pour réduire son indication de 1 dans le cas où le discriminateur de Wenckebach a détecté que deux cycles ventriculaires actuels et consécutifs diffèrent de plus d'une cote de différence prédéfinie de la valeur moyenne de la durée de cycle ou n'a pas généré de signal de Wenckebach pour une pluralité d'au moins cinq cycles ventriculaires consécutifs et actuels.

10. Détecteur d'audiofréquence selon la revendication 9, l'unité d'analyse étant conçue pour effacer le signal de suspicion d'audiofréquence et pour remettre à zéro le compteur lorsque l'indication du compteur dépasse un seuil d'indication de compteur prédéfini.

11. Détecteur d'audiofréquence selon l'une quelconque des revendications 1 à 10, le détecteur d'audiofréquence étant un composant d'un appareil médical implantable.

12. Détecteur d'audiofréquence selon la revendication 11, l'appareil médical implantable étant un pacemaker ou un cardioverteur/ défibrillateur.
